# EUROPÄISCHE PATENTANMELDUNG

(11) **EP 1 270 720 A2**
(43) Veröffentlichungstag der Anmeldung: **02.01.2003**
(21) Anmeldenummer: 02012593.6
(22) Anmeldetag: 06.06.2002
(51) Int. Cl.: C12N 9/86, C12N 15/55, C12N 5/10

(54) **Aktivierte rec-D-Hydantoinasen**

(30) Priorität: 22.06.2001 DE 10130169
(71) Anmelder: Degussa AG, 40474 Düsseldorf (DE)
(72) Erfinder: Bommarius, Andreas, Dr., 30327 Atlanta (US); Drauz, Karlheinz, Prof., 63517 Freigericht (DE); May, Oliver, Dr., 60388 Frankfurt am Main (DE); Siemann-Herzberg, Martin, Dr., 72218 Wildberg (DE); Syldatk, Christph, Prof. Dr., 70565 Stuttgart (DE); Werner, Markus, 91056 Erlangen (DE); Altenbuchner, Josef, Dr., 71154 Nufringen (DE)

(57) **Zusammenfassung**

Die vorliegende Erfindung richtet sich auf rec-Hydantoinasen, welche durch ein bestimmtes Verfahren in aktiverer Form erhalten werden können. U.a. betrifft die Erfindung auch eine rec-Hydantoinase aus dem Organismus Arthrobacter crystallopoietes DSM20117, Nukleinsäuren, welche für ein derartiges Protein codieren und die sie enthaltenenden Vehikel sowie deren Verwendungen.

SEQUENZPROTOKOLL

<110> Degussa AG

<120> D-Hydantoinase aus Arthrobacter crystallopoietes DSM
      20117

<130> 010141 AM

<140>
<141>

<160> 25

<170> PatentIn Ver. 2.1

<210> 1
<211> 1314
<212> DNA
<213> Arthrobacter crystallopoietes

<220>
<221> CDS
<222> (1)..(1314)

<400> 1
atg gat gcg aaa ctc ctt gtt ggc ggc act att gtt tcc tcg acc ggc    48
Met Asp Ala Lys Leu Leu Val Gly Gly Thr Ile Val Ser Ser Thr Gly
  1               5                  10                  15

aaa atc cga gcc gac gtg ctg att gaa aac ggc aaa gtc gcc gct gtc    96
Lys Ile Arg Ala Asp Val Leu Ile Glu Asn Gly Lys Val Ala Ala Val
             20                  25                  30

ggc atg ctg gac gcc gcg acg ccg gac aca gtt gag cgg gtt gac tgc    144
Gly Met Leu Asp Ala Ala Thr Pro Asp Thr Val Glu Arg Val Asp Cys
         35                  40                  45

gac ggc aaa tac gtc atg ccc ggc ggt atc gac gtt cac acc cac atc    192
Asp Gly Lys Tyr Val Met Pro Gly Gly Ile Asp Val His Thr His Ile
     50                  55                  60

gac tcc ccc ctc atg ggg acc acc acc gcc gat gat ttt gtc agc gga    240
Asp Ser Pro Leu Met Gly Thr Thr Thr Ala Asp Asp Phe Val Ser Gly
 65                  70                  75                  80

acg att gca gcc gct acc ggc gga aca acg acc atc gtc gat ttc gga    288
Thr Ile Ala Ala Ala Thr Gly Gly Thr Thr Thr Ile Val Asp Phe Gly
                 85                  90                  95

cag cag ctc gcc ggc aag aac ctg ctg gaa tcc gca gac gcg cac cac    336
Gln Gln Leu Ala Gly Lys Asn Leu Leu Glu Ser Ala Asp Ala His His
            100                 105                 110

aaa aag gcg cag ggg aaa tcc gtc att gat tac ggc ttc cat atg tgc    384
Lys Lys Ala Gln Gly Lys Ser Val Ile Asp Tyr Gly Phe His Met Cys
        115                 120                 125

gtg acg aac ctc tat gac aat ttc gat tcc cat atg gca gaa ctg aca    432

Val Thr Asn Leu Tyr Asp Asn Phe Asp Ser His Met Ala Glu Leu Thr
    130                 135                 140

cag gac gga atc tcc agt ttc aag gtc ttc atg gcc tac cgc gga agc     480
Gln Asp Gly Ile Ser Ser Phe Lys Val Phe Met Ala Tyr Arg Gly Ser
145                 150                 155                 160

ctg atg atc aac gac ggc gaa ctg ttc gac atc ctc aag gga gtc ggc     528
Leu Met Ile Asn Asp Gly Glu Leu Phe Asp Ile Leu Lys Gly Val Gly
                165                 170                 175

tcc agc ggt gcc aaa cta tgc gtc cac gca gag aac ggc gac gtc atc     576
Ser Ser Gly Ala Lys Leu Cys Val His Ala Glu Asn Gly Asp Val Ile
            180                 185                 190

gac agg atc gcc gcc gac ctc tac gcc caa gga aaa acc ggg ccc ggg     624
Asp Arg Ile Ala Ala Asp Leu Tyr Ala Gln Gly Lys Thr Gly Pro Gly
        195                 200                 205

acc cac gag atc gca cgc ccg ccg gaa tcg gaa gtc gaa gca gtc agc     672
Thr His Glu Ile Ala Arg Pro Pro Glu Ser Glu Val Glu Ala Val Ser
    210                 215                 220

cgg gcc atc aag atc tcc cgg atg gcc gag gtg ccg ctg tat ttc gtg     720
Arg Ala Ile Lys Ile Ser Arg Met Ala Glu Val Pro Leu Tyr Phe Val
225                 230                 235                 240

cat ctt tcc acc cag ggg gcc gtc gag gaa gta gct gcc gcg cag atg     768
His Leu Ser Thr Gln Gly Ala Val Glu Glu Val Ala Ala Ala Gln Met
                245                 250                 255

aca gga tgg cca atc agc gcc gaa acg tgc acc cac tac ctg tcg ctg     816
Thr Gly Trp Pro Ile Ser Ala Glu Thr Cys Thr His Tyr Leu Ser Leu
            260                 265                 270

agc cgg gac atc tac gac cag ccg gga ttc gag ccg gcc aaa gct gtc     864
Ser Arg Asp Ile Tyr Asp Gln Pro Gly Phe Glu Pro Ala Lys Ala Val
        275                 280                 285

ctc aca cca ccg ctg cgc aca cag gaa cac cag gac gcg ttg tgg aga     912
Leu Thr Pro Pro Leu Arg Thr Gln Glu His Gln Asp Ala Leu Trp Arg
    290                 295                 300

ggc att aac acc ggt gcg ctc agc gtc gtc agt tcc gac cac tgc ccc     960
Gly Ile Asn Thr Gly Ala Leu Ser Val Val Ser Ser Asp His Cys Pro
305                 310                 315                 320

ttc tgc ttt gag gaa aag cag cgg atg ggg gca gat gac ttc cgg cag     1008
Phe Cys Phe Glu Glu Lys Gln Arg Met Gly Ala Asp Asp Phe Arg Gln
                325                 330                 335

atc ccc aac ggc ggg ccc ggc gtg gag cac cga atg ctc gtg atg tat     1056
Ile Pro Asn Gly Gly Pro Gly Val Glu His Arg Met Leu Val Met Tyr
            340                 345                 350

gag acc ggt gtc gcg gaa gga aaa atg acg atc gag aaa ttc gtc gag     1104
Glu Thr Gly Val Ala Glu Gly Lys Met Thr Ile Glu Lys Phe Val Glu
        355                 360                 365

gtg act gcc gag aac ccg gcc aag caa ttc gat atg tac ccg aaa aag    1152
Val Thr Ala Glu Asn Pro Ala Lys Gln Phe Asp Met Tyr Pro Lys Lys
    370                 375                 380

gga aca att gca ccg ggc tcc gat gca gac atc atc gtg gtc gac ccc    1200
Gly Thr Ile Ala Pro Gly Ser Asp Ala Asp Ile Ile Val Val Asp Pro
385                 390                 395                 400

aac gga aca acc ctc atc agt gcc gac acc caa aaa caa aac atg gac    1248
Asn Gly Thr Thr Leu Ile Ser Ala Asp Thr Gln Lys Gln Asn Met Asp
                405                 410                 415

tac acg ctg ttc gaa ggc ttc aaa atc cgt tgc tcc atc gac cag gtg    1296
Tyr Thr Leu Phe Glu Gly Phe Lys Ile Arg Cys Ser Ile Asp Gln Val
            420                 425                 430

ttc tcg cgt ggc gac ctg                                            1314
Phe Ser Arg Gly Asp Leu
        435

<210> 2
<211> 438
<212> PRT
<213> Arthrobacter crystallopoietes

<400> 2
Met Asp Ala Lys Leu Leu Val Gly Gly Thr Ile Val Ser Ser Thr Gly
  1               5                  10                  15

Lys Ile Arg Ala Asp Val Leu Ile Glu Asn Gly Lys Val Ala Ala Val
             20                  25                  30

Gly Met Leu Asp Ala Ala Thr Pro Asp Thr Val Glu Arg Val Asp Cys
         35                  40                  45

Asp Gly Lys Tyr Val Met Pro Gly Gly Ile Asp Val His Thr His Ile
     50                  55                  60

Asp Ser Pro Leu Met Gly Thr Thr Thr Ala Asp Asp Phe Val Ser Gly
 65                  70                  75                  80

Thr Ile Ala Ala Ala Thr Gly Gly Thr Thr Thr Ile Val Asp Phe Gly
                 85                  90                  95

Gln Gln Leu Ala Gly Lys Asn Leu Leu Glu Ser Ala Asp Ala His His
            100                 105                 110

Lys Lys Ala Gln Gly Lys Ser Val Ile Asp Tyr Gly Phe His Met Cys
        115                 120                 125

Val Thr Asn Leu Tyr Asp Asn Phe Asp Ser His Met Ala Glu Leu Thr
    130                 135                 140

Gln Asp Gly Ile Ser Ser Phe Lys Val Phe Met Ala Tyr Arg Gly Ser
145                 150                 155                 160

Leu Met Ile Asn Asp Gly Glu Leu Phe Asp Ile Leu Lys Gly Val Gly
                165                 170                 175

Ser Ser Gly Ala Lys Leu Cys Val His Ala Glu Asn Gly Asp Val Ile
            180                 185                 190

Asp Arg Ile Ala Ala Asp Leu Tyr Ala Gln Gly Lys Thr Gly Pro Gly
        195                 200                 205

Thr His Glu Ile Ala Arg Pro Pro Glu Ser Glu Val Glu Ala Val Ser
    210                 215                 220

Arg Ala Ile Lys Ile Ser Arg Met Ala Glu Val Pro Leu Tyr Phe Val
225                 230                 235                 240

His Leu Ser Thr Gln Gly Ala Val Glu Glu Val Ala Ala Ala Gln Met
                245                 250                 255

Thr Gly Trp Pro Ile Ser Ala Glu Thr Cys Thr His Tyr Leu Ser Leu
            260                 265                 270

Ser Arg Asp Ile Tyr Asp Gln Pro Gly Phe Glu Pro Ala Lys Ala Val
        275                 280                 285

Leu Thr Pro Pro Leu Arg Thr Gln Glu His Gln Asp Ala Leu Trp Arg
    290                 295                 300

Gly Ile Asn Thr Gly Ala Leu Ser Val Val Ser Ser Asp His Cys Pro
305                 310                 315                 320

Phe Cys Phe Glu Glu Lys Gln Arg Met Gly Ala Asp Asp Phe Arg Gln
                325                 330                 335

Ile Pro Asn Gly Gly Pro Gly Val Glu His Arg Met Leu Val Met Tyr
            340                 345                 350

Glu Thr Gly Val Ala Glu Gly Lys Met Thr Ile Glu Lys Phe Val Glu
        355                 360                 365

Val Thr Ala Glu Asn Pro Ala Lys Gln Phe Asp Met Tyr Pro Lys Lys
    370                 375                 380

Gly Thr Ile Ala Pro Gly Ser Asp Ala Asp Ile Ile Val Val Asp Pro
385                 390                 395                 400

Asn Gly Thr Thr Leu Ile Ser Ala Asp Thr Gln Lys Gln Asn Met Asp
                405                 410                 415

Tyr Thr Leu Phe Glu Gly Phe Lys Ile Arg Cys Ser Ile Asp Gln Val
            420                 425                 430

Phe Ser Arg Gly Asp Leu
        435

<210> 3
<211> 26
<212> DNA
<213> Künstliche Sequenz

<220>
<223> Beschreibung der künstlichen Sequenz:IPCR1+

<400> 3
gatgttcacg caccttcttt cacttc                                          26


<210> 4
<211> 25
<212> DNA
<213> Künstliche Sequenz

<220>
<223> Beschreibung der künstlichen Sequenz:IPCR1-

<400> 4
ggtgttgtag cccaggacga cgagc                                           25


<210> 5
<211> 26
<212> DNA
<213> Künstliche Sequenz

<220>
<223> Beschreibung der künstlichen Sequenz:IPCR5+

<400> 5
gagggcgatg aagtcgtcgt tgtgaa                                          26


<210> 6
<211> 25
<212> DNA
<213> Künstliche Sequenz

<220>
<223> Beschreibung der künstlichen Sequenz:IPCR5-

<400> 6
gttctggtat gcccctgcct gaagt                                           25


<210> 7
<211> 26
<212> DNA
<213> Künstliche Sequenz

<220>
<223> Beschreibung der künstlichen Sequenz:K_DCn2

<400> 7
aacatatggc gaaaaacttg atgctc                                          26


<210> 8
<211> 26
<212> DNA
<213> Künstliche Sequenz

<220>
<223> Beschreibung der künstlichen Sequenz:K_DCc2

<400> 8
aaggatccgt cattcacgtt gaacgg                                          26


<210> 9
<211> 29
<212> DNA
<213> Künstliche Sequenz

<220>
<223> Beschreibung der künstlichen Sequenz:K_DCc3

<400> 9
aaggatcctt agtcattcac gttgaacgg                                       29


<210> 10
<211> 17
<212> DNA
<213> Künstliche Sequenz

<220>
<223> Beschreibung der künstlichen Sequenz:61.61a

<400> 10
gtnatgtayg aracvgg                                                    17


<210> 11
<211> 17
<212> DNA
<213> Künstliche Sequenz

<220>
<223> Beschreibung der künstlichen Sequenz:73.31b

<400> 11
gtrtartcca trttytc                                                    17


<210> 12
<211> 13
<212> PRT
<213> Künstliche Sequenz

<220>
<223> Beschreibung der künstlichen Sequenz:61.61_prt

<400> 12
Ser Leu Val Met Tyr Glu Thr Gly Val Ala Glu Gly Lys
  1               5                  10


<210> 13
<211> 11

<212> PRT
<213> Künstliche Sequenz

<220>
<223> Beschreibung der künstlichen Sequenz:73.31_prt

<400> 13
Gln Asn Met Asp Tyr Thr Leu Phe Glu Gly Lys
1               5                   10

<210> 14
<211> 23
<212> DNA
<213> Künstliche Sequenz

<220>
<223> Beschreibung der künstlichen Sequenz:IPCR7+

<400> 14
atcgtggtcg accccaacgg aac                                          23

<210> 15
<211> 24
<212> DNA
<213> Künstliche Sequenz

<220>
<223> Beschreibung der künstlichen Sequenz:IPCR7-

<400> 15
gcatcggagc ccggtgcaat tgtt                                         24

<210> 16
<211> 22
<212> DNA
<213> Künstliche Sequenz

<220>
<223> Beschreibung der künstlichen Sequenz:IPCR11+

<400> 16
atgcggtcgc aaccacaacc ca                                           22

<210> 17
<211> 22
<212> DNA
<213> Künstliche Sequenz

<220>
<223> Beschreibung der künstlichen Sequenz:IPCR11-

<400> 17
agcgccaggg ccggaagaag ca                                           22

<210> 18
<211> 19
<212> DNA
<213> Künstliche Sequenz

<220>
<223> Beschreibung der künstlichen Sequenz:S1995

<400> 18
ggcccatttt cctgtcagt                                                    19


<210> 19
<211> 18
<212> DNA
<213> Künstliche Sequenz

<220>
<223> Beschreibung der künstlichen Sequenz:S998

<400> 19
aggctgaaaa tcttctct                                                     18


<210> 20
<211> 28
<212> DNA
<213> Künstliche Sequenz

<220>
<223> Beschreibung der künstlichen Sequenz:K_LCn1

<400> 20
aacatatgga aacaattgac ggcatttc                                          28


<210> 21
<211> 26
<212> DNA
<213> Künstliche Sequenz

<220>
<223> Beschreibung der künstlichen Sequenz:K_LCc1

<400> 21
aaggatccgg gccgtgactc gtcgac                                            26


<210> 22
<211> 45
<212> DNA
<213> Künstliche Sequenz

<220>
<223> Beschreibung der künstlichen Sequenz:K_DHPn2

<400> 22
aaaaggatcc gaaggagata tacaatggan gcgaaactcc ttgtt                       45

<210> 23
<211> 33
<212> DNA
<213> Künstliche Sequenz

<220>
<223> Beschreibung der künstlichen Sequenz:K_DHPc2

<400> 23
aaaaaagctt ctaccgcttg atgaattcgc cgc 33

<210> 24
<211> 61
<212> DNA
<213> Künstliche Sequenz

<220>
<223> Beschreibung der künstlichen Sequenz:K_DHPc5

<400> 24
aaaagctttt atttttcgaa ctgcgggtcg ctccaagcgc tccgcttgat gaattcgccc 60
g 61

<210> 25
<211> 26
<212> DNA
<213> Künstliche Sequenz

<220>
<223> Beschreibung der künstlichen Sequenz:K_DCn1

<400> 25
aacatatgct cgcggtcgct caagtc 26

## Beschreibung

Die vorliegende Erfindung richtet sich auf rec-Hydantoinasen, welche durch ein bestimmtes Verfahren in aktiverer Form erhalten werden können. U.a. betrifft die Erfindung auch eine rec-Hydantoinase aus dem Organismus Arthrobacter crystallopoietes DSM20117, Nukleinsäuren, welche für ein derartiges Protein codieren und die sie enthaltenden Vehikel.

Der Einsatz enzymatischer Verfahren in der Synthese von organischen Verbindungen ist im großtechnischen Maßstab wohl etabliert, da diese in Bezug auf die Selektivitäten und Ausbeuten an Produkten den normalen chemischen Verfahren häufig überlegen sind. Gerade enantiomerenangereicherte Aminosäuren sind bevorzugte Targets für den Einsatz enzymatisch arbeitender Verfahren, haben diese Prozesse doch auch in der Natur eine entscheidende Bedeutung z.B. bei der Biosynthese von Aminosäuren, Proteinen und Eiweißen. Enantiomerenangereicherte Aminosäuren sind weiterhin wichtige Produkte im Hinblick auf die Synthese bioaktiver Verbindungen oder bei der parenteralen Ernährung.

Hydantoinasen sind Enzyme, welche befähigt sind, 5'-substituierte Hydantoine ggf. stereoselektiv in die L- oder D-N-Carbamoylaminosäure umzusetzen (Schema 1).

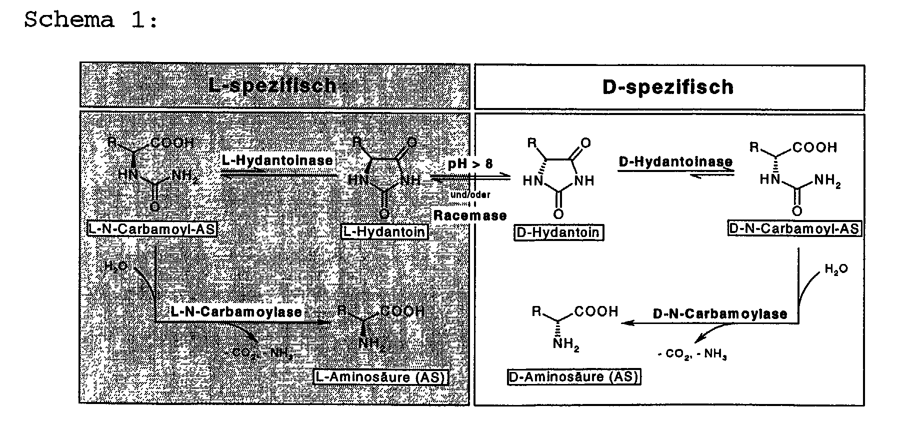

Die racemischen 5'-substituierten Hydantoine lassen sich vorzugsweise recht einfach durch chemische Synthesen (Kleinpeter, Structural Chemistry 1997, 8, 161-173; Ogawa et al., Tibtech 1999, 17, 1039-43; Beller et al., Angew. Chem. 1999, 111, 1562-65) gewinnen. Die anvisierten Verfahren zur Herstellung von enantiomerenangereicherten Aminosäuren finden deshalb vorzugsweise im technischen Maßstab Anwendung (Drauz K, Kottenhahn M, Makryaleas K, Klenk H, Bernd M, Angew Chem, (1991). Chemoenzymatic synthesis of D-ω-ureidoaminoacids, 103, 704-706.; Schema 2).

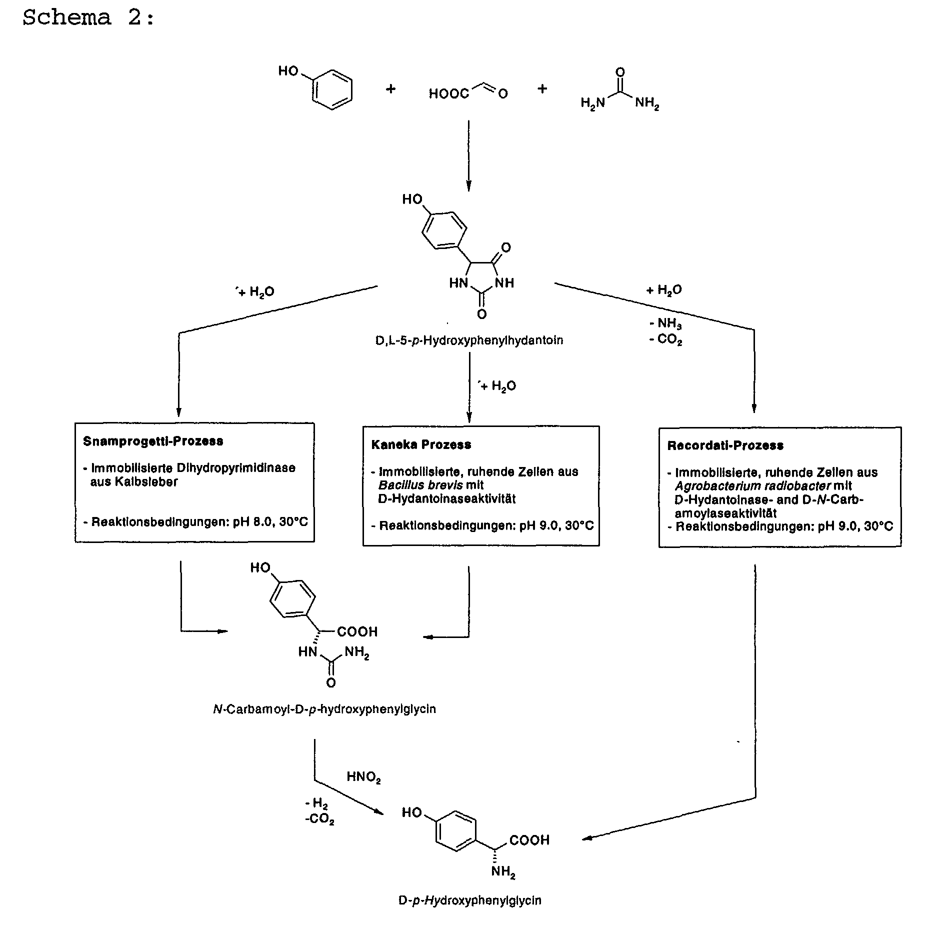

Beim Screening nach Hydantoin-verwertenden Mikroorganismen wurden bisher sowohl Gram-positive als auch Gram-negative Prokaryonten aus fünf verschiedenen phylogenetischen Gruppen isoliert: Alcaligenes, Arthrobacter, Bacillus, Blastobacter, Flavobacterium, Nocardia, Pseudomonas, Comamonas, Thermus und Agrobacterium. Eine vollständige Anordnung der Gene, die für die am Abbau beteiligten Enzyme kodieren, ist bisher nur für drei Organismen beschrieben worden. Dabei sind die codierenden Strukturgene der Enzyme in Form eines sogenannten hyu-Genclusters (hyu steht für "hydantoin utilizing"; nach Watabe et al., J. Bacteriol. 1992, 174, 3461-66; ibid, 962-969) auf der genomischen DNA (Arthrobacter und Agrobacterium) oder auf einem Plasmid (Pseudomonas) nebeneinander angeordnet. Von den drei hyu-Genclustern beinhalten Agrobacterium und Pseudomonas die Gene für eine D-selektive Spaltung und Arthrobacter Gene für eine L-selektive Spaltung (Hils, Dissertation Universität Stuttgart, 1998; Watabe et al., s.o.; Wiese, Dissertation Universität Stuttgart, 2000, Verlag Ulrich Grauer).

Bekannt war ebenfalls, daß der Organismus Arthrobacter crystallopoietes DSM 20117 über eine D-Hydantoinase verfügt (Syldatk et al. in Jahrbuch Biotechnologie, Band 2, 1988/1989, Ed.: P. Präve). Die n-terminale Sequenz des Enzyms konnte bereits aufgeklärt werden (A. Marin, Dissertation Universität Stuttgart, 1997).

Bisher konnte die Herstellung der beschriebenen Hydantoinase in rekombinanter und aktiver Form jedoch nicht bewerkstelligt werden (M. Werner Dissertation, Universität Stuttgart 2001).

Aufgabe der vorliegenden Erfindung ist die Angabe eines Verfahrens zur rekombinanten Herstellung von aktiven Hydantoinasen sowie die Angabe der nach diesem Verfahren gewonnen aktiven rekombinanten Hydantoinasen.

Die Aufgabe wird anspruchsgemäß gelöst. Anspruch 1 bezieht sich auf ein Verfahren zur rekombinanten Herstellung von Hydantoinasen. Ansprüche 2 und 3 beziehen sich auf bevorzugte Ausführungsformen für das erfindungsgemäße Verfahren. Anspruch 4 schützt die so erhältlichen rec-Hydantoinasen, während Anspruch 5 auf die dieses Enzym codierenden Nukleinsäuren gerichtet sind. Anspruch 6 wiederum betrifft Vehikel, welche die erfindungsgemäßen Nukleinsäuren aufweisen, Anspruch 7 ist auf Nukleinsäuren gerichtet, welche mit den erfindungsgemäßen Nukleinsäuren hybridisieren, Anspruch 8 schützt bevorzugte Primer. Anspruch 9 ist auf ein Verfahren zur Verbesserung der Hydantoinasen ausgehend von erfindungsgemäßen Nukleinsäuren gerichtet, wohingegen Anspruch 10 bestimmte verbesserte rec-Hydantoinasen umfaßt. Anspruch 11 und 12 richten sich auf bestimmte Verwendungen der rec-Hydantoinasen bzw. erfindungsgemäßen Nukleinsäuren.

Dadurch, daß in einem Verfahren zur Herstellung aktivierter rec-Hydantoinasen durch Fermentation der die rec-Hydantoinasen bildenden Mikroorganismen in Gegenwart zweiwertiger Metallionen die Fermentationsbrühe eine die Aktivierung bedingende Konzentration an Metallionen (z.B. Co, Mn, Zn) aufweist, gelangt man in überraschend einfacher dafür aber nicht minder vorteilhafter Art und Weise zu rec-Hydantoinasen, die gegenüber den rec-Hydantoinasen, die von den unter den sonst üblichen Bedingungen fermentierten die rec-Hydantoinasen expremierenden Mikroorganismen gebildet werden, eine erhöhte spezifische Aktivität (Aktivierung) aufweisen. Es ist als überraschend zu werten, daß insbesondere die Erhöhung der Konzentration an Zinkionen, welche eigentlich für das Wachstum der Mikroorganismen während der Fermentation nachteilig ist (längere Wachstumsdauer wird benötigt), zu einer beträchtlichen Aktivitätssteigerung führt.

Vorzugsweise weist also die Fermentationsbrühe eine die Aktivitätssteigerung bedingende Konzentration an Zinkionen auf. Die optimale Konzentration, in der die Metallionen, insbesondere die Zinkionen zur Fermentationsbrühe zugegeben werden, kann vom Fachmann anhand von Routineexperimenten ermittelt werden. Sie sollte einerseits so hoch gewählt werden, daß eine erfindungsgemäße Aktivierung/Aktivitätssteigerung zu erzielen ist, andererseits sollte sie nicht so hoch liegen, daß das Wachstum der Mikroorganismen über die Maßen gehemmt wird, ohne einen weiteren Aktivitätszuwachs zu kreieren. Vorzugsweise wird die Konzentration an Metallionen, z.B. Zinkionen bei der Fermentation auf ≥30 µmol/l, besonders bevorzugt auf ≥50 µmol/l und äußerst bevorzugt auf ≥80 µmol/l gesteigert.
Besonders bevorzugt handelt es sich bei der betrachteten rec-Hydantoinase um die Hydantoinase aus Arthrobacter crystallopoietes DSM20117.

In einer weiteren Ausgestaltung beschäftigt sich die Erfindung mit rec-Hydantoinasen erhältlich nach dem erfindungsgemäßen Verfahren. Es ist davon auszugehen, daß, obwohl die Primärstruktur der aktivierten und nicht aktivierten Enzyme übereinstimmt, die Erhöhung der Zinkionen-Konzentration während der Fermentation vermutlich einen Einfluß auf die sich bildende Sekundär- oder gar Tertiärstruktur der Enzyme dergestalt besitzt, daß eine Verbesserung der spezifischen Aktivität der Proteine erzielt wird.

In einer nächsten Ausgestaltung beschäftigt sich die Erfindung mit Nukleinsäuren codierend für eine D-Hydantoinase aus Arthrobacter crystallopoietes DSM 20117.

Durch die Angabe der Nukleinsäuren codierend für eine D-Hydantoinase aus Arthrobacter crystallopoietes DSM 20117, gelangt man in vorteilhafter Art und Weise zu Substanzen, welche es erlauben, die für einen enzymatisch-technischen Prozeß zur Erzeugung von D-Aminosäuren notwendigen Enzyme in ausreichender Menge sicherzustellen. Über rekombinante Techniken ist es mit den Nukleinsäuren möglich, die Enzyme aus schnell wachsenden Wirtsorganismen in hohen Ausbeuten zu gewinnen. Außerdem sind die erfindungsgemäßen Gensequenzen zur Erzeugung von verbesserten Hydantoinasemutanten zu verwenden.

In einer nächsten Ausgestaltung bezieht sich die Erfindung auf Plasmide oder Vektoren aufweisend eine oder mehrere der erfindungsgemäßen Nukleinsäuren.

Als Plasmide oder Vektoren kommen im Prinzip alle dem Fachmann für diesen Zweck zur Verfügung stehenden Ausführungsformen in Frage. Derartige Plasmide und Vektoren können Studier et al., Methods Enzymol. 1990, 185, 61-69 oder den Broschüren der Firmen Novagen, Promega, New England Biolabs, Clontech oder Gibco BRL entnommen werden. Weiter bevorzugte Plasmide und Vektoren können gefunden werden in: DNA cloning: a practical approach. Volume I-III, edited by D. M. Glover, IRL Press Ltd., Oxford, Washington DC, 1985, 1987; Denhardt, D. T. and Colasanti, J.: A surey of vectors for regulating expression of cloned DNA in E. coli. In: Rodriguez, R.L. and Denhardt, D. T (eds), Vectors, Butterworth, Stoneham, MA, 1987, pp179-204; Gene expression technology. In: Goeddel, D. V. (eds), Methods in Enzymology, Volume 185, Academic Press, Inc., San Diego, 1990; Sambrook, J., Fritsch, E.F. and Maniatis, T. 1989. Molecular cloning: a laboratory manual, 2^{nd} ed. Cold Spring Harbor Laboratory Press, Cold Spring Harbor, N.Y.
Plasmide, mit denen das die erfindungsgemäße Nukleinsäure aufweisende Genkonstrukt in ganz bevorzugter Weise in den Wirtsorganismus kloniert werden kann, sind: pKK-177-3H (Roche Biochemicals), pBTac (Roche Biochemicals), pKK-233 (Stratagene) oder pET (Novagen). Mit Außnahme der TOPO-Serie, die eine Kanamycin-Resistenz integriert hat, sollten alle anderen Plasmide eine β-Lactamase für die Ampicilin-Resistenz enthalten. Äußerst bevorzugte Plasmide sind die folgenden:

| **Bezeichnung** | **Eigenschaften** | **beteiligte Primer** |
|---|---|---|
| pJW2 | pCRTOPOBluntII (Fig. 1) mit Amplikon aus IPCR 1 | IPCR1+/- |
| pRW | pCRTOPOBluntII mit Amplikon aus IPCR 2 | IPCR5+/5- |
| pMW1 | pJOE4036 (Fig. 2) + DC (ttg-Start) mit His-tag | K_DCn2/c2 |
| pMW2 | pJOE4036 + DC (ttg-Start) ohne His-tag | K_DCn2/c3 |
| pMW3 | pJOE4036 + DC (atg-Start) mit His-tag | K_DCn1/c2 |
| pMW10 | pJOE3078 (Fig. 3) + DHP mit Strep-tag | K_DHPn2/c5 |
| pMW11 | pJOE3078 + DHP ohne Strep-tag | K_DHPn2/c2 |
| pJW1 | pCRTOPOBluntII mit Hydantoinasefragment aus PCR mit degenerierten Primern | 51.61a/73.31b |
| pCF1 | pCRTOPOBluntII mit Amplikon aus IPCR 3 | IPCR1+/- |
| PCF2 | pCRTOPOBluntII mit Amplikon aus IPCR 4 | IPCR1+/- |

Gleichfalls ist die Erfindung auf Mikroorganismen aufweisend die erfindungsgemäßen Nukleinsäuren gerichtet.

Der Mikroorganismus, in den die Nukleinsäuren kloniert wird, dient zur Vermehrung und Gewinnung einer ausreichenden Menge des rekombinanten Enzyms. Die Verfahren hierfür sind dem Fachmann wohlbekannt (Sambrook et al. 1989, Molecular cloning: A Laboratory Manual, 2nd Edition, Cold Spring Harbor Laboratory Press, Balbas P & Bolivar F. 1990, Design and construction of expression plasmd vectors in E.coli, Methods Enzymology 185, 14-37). Als Mikroorganismen können im Prinzip alle dem Fachmann für diesen Zweck in Frage kommenden Organismen herangezogen werden. Vorzugsweise sind E. coli-Stämme für diesen Zweck zu benutzen. Ganz besonders bevorzugt sind: E. coli NM 522, JM109, JM105, RR1, DH5α, TOP 10⁻ oder HB101. Plasmide, mit denen das die erfindungsgemäße Nukleinsäure aufweisende Genkonstrukt vorzugsweise in den Wirtsorganismus kloniert wird, sind weiter oben angegeben.

Mitumfaßt von der Erfindung sind auch Nukleinsäuren, welche unter stringenten Bedingungen mit den erfindungsgemäßen einzelsträngigen Nukleinsäuren oder deren komplementären einzelsträngigen Nukleinsäuren hybridisieren.
Der Ausdruck "unter stringenten Bedingungen" wird hierin wie bei Sambrook et al. (Molecular Cloning, A Laboratory Manual, Cold Spring Harbor Laboratory Press (1989), 1.101-1.104) beschrieben, verwendet. Bevorzugt liegt eine stringente Hybridisierung gemäß der vorliegenden Erfindung vor, wenn nach Waschen für eine Stunde mit 1 x SSC und 0,1 % SDS (Natriumdodecylsulfonat) bei 50 °C, bevorzugt bei 55 °C, mehr bevorzugt bei 62 °C und am meisten bevorzugt bei 68 °C und mehr bevorzugt für 1 Stunde mit 0,2 x SSC und 0,1 % SDS bei 50 °C, bevorzugter bei 55 °C, mehr bevorzugt bei 62 °C und am meisten bevorzugt bei 68 °C noch ein positives Hybridisierungssignal beobachtet wird.

Ein folgender Aspekt der Erfindung richtet sich auf Primer zur Herstellung der erfindungsgemäßen Gensequenzen mittels aller Arten von PCR. Mitumfaßt sind die Sense- und Antisense-Primer codierend für die entsprechenden Aminosäuresequenzen.
Geeignete Primer können prinzipiell nach dem Fachmann bekannten Verfahren gewonnen werden. Das Auffinden der erfindungsgemäßen Primer erfolgt durch Vergleich mit bekannten DNA-Sequenzen oder durch Übersetzung der ins Auge gefaßten Aminosäuresequenzen in das Codon des betrachteten Organismus (z.B. für Streptomyces: Wright et al., Gene 1992, 113, 55-65). Gemeinsamkeiten in der Aminosäuresequenz von Proteinen von sogenannten Superfamilien ist hierfür ebenfalls von Nutzen (Firestine et al., Chemistry & Biology 1996, 3, 779-783). Weitere Informationen diesbezüglich können gefunden werden in Oligonucleotide synthesis: a practical approach, edited by M.J. Gait, IRL Press Ltd, Oxford Washington DC, 1984; PCR Protocols: A guide to methods and applications, edited by M.A. Innis, D.H. Gelfound, J.J. Sninsky and T.J. White. Academic Press, Inc., San Diego, 1990. Äußerst bevorzugt sind folgende Primer:

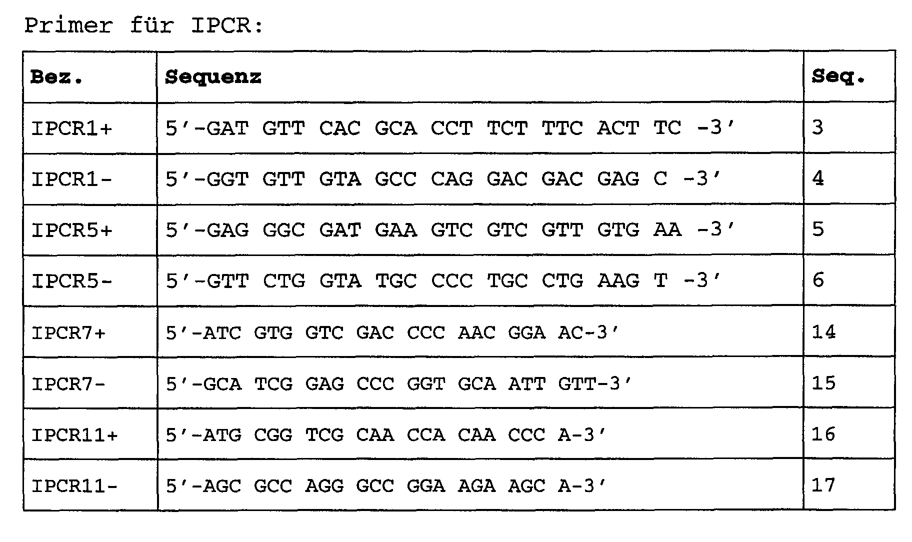

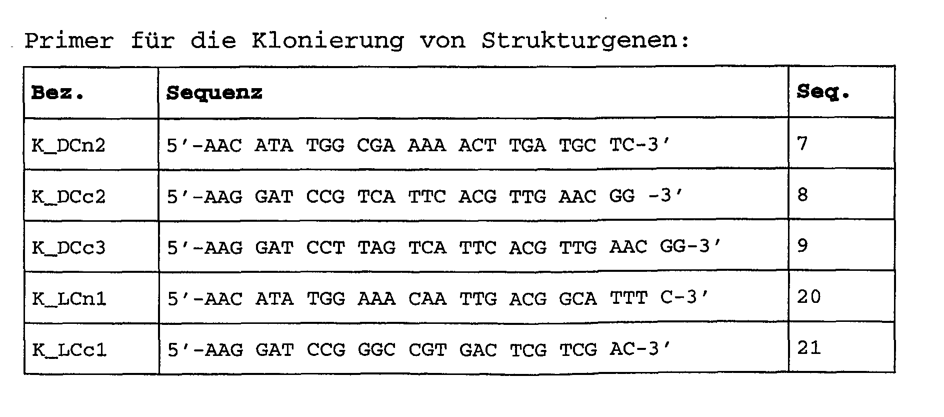

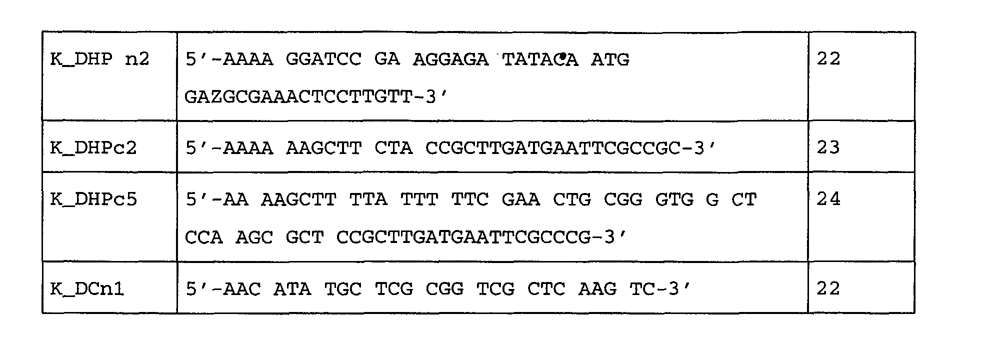

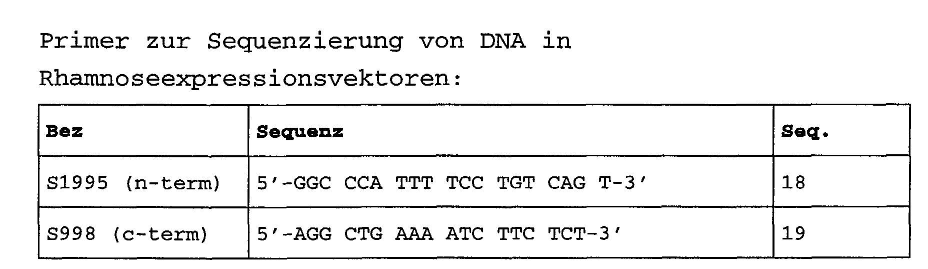

In einer wiederum nächsten Ausgestaltung bezieht sich die vorliegende Erfindung auf ein Verfahren zur Herstellung verbesserter rec-Hydantoinasen und derart gewonnene rec-Hydantoinasen oder diese codierende Nukleinsäuren, wobei man ausgehend von den erfindungsgemäßen Nukleinsäuren codierend für eine rec-Hydantoinase,
a) die Nukleinsäuren einer Mutagense unterwirft,
b) die aus a) erhältlichen Nukleinsäuren in einen geeigneten Vektor kloniert und diesen in ein geeignetes Expressionsystem transferiert und
c) die gebildeten Proteine mit verbesserter Aktivität und/oder Selektivität detektiert und isoliert.

Die Vorgehensweise zur Verbesserung der erfindungsgemäßen Enzyme durch Mutagenese-Methoden ist dem Fachmann hinlänglich bekannt. Als Mutagenese-Methoden kommen alle dem Fachmann für diesen Zweck zur Verfügung stehenden Methoden in Frage. Insbesondere sind dies die Sättigungsmutagenese, die Random-Mutagenesis, Shuffling-Methoden sowie Site-Directed-Mutagenesis (Lit. s.u.). Die erhaltenen neuen Nukleinsäuresequenzen werden nach den weiter oben dargestellten Methoden in einen Wirtsorgansmus (Lit. s.o.) kloniert und die expremierten Enzyme mit geeigneten Screening-Methoden (Roberts J., Stella V.J. and Decedue C.J. (1985) A colorimetric assay of pancreatic lipase: rapid detection of lipase and colipase separated by gel filtration. Lipids **20**(1): 42-45; Pratt R.F., Faraci W.S. and Govardhan C.P. (1985) A direct spectrophotometric assay for D-alanine carboxypeptidases and for the esterase activity of beta-lactamases. Anal. Biochem. **144**(1): 204-206; Brückner, H., R. Wittner, and H. Godel (1991) Fully automated high-performance liquid chromatographic separation of DL-amino acids derivatized with o-Phthaldialdehyde together with N-isopropyl-cysteine. Application to food samples) detektiert.

Die vorliegende Erfindung beschäftigt sich ebenfalls mit der Verwendung der erfindungsgemäßen ggf. durch Mutation verbesserten rec-Hydantoinasen zur Herstellung von N-Carbamoylaminosäuren bzw. Aminosäuren.

Weiterhin eigenen sich die erfindungsgemäßen und darüberhinaus weiter verbesserten Nukleinsäuren, die für die betrachteten rec-Hydantoinasen codieren, bevorzugt zur Herstellung von Ganzzellkatalysatoren (DE10037115.9 sowie dort zitierte Lit.).

Die erfindungsgemäßen Nukleinsäuren lassen sich also vorzugsweise zur Herstellung von rec-Hydantoinasen einsetzen. Durch rekombinante Techniken, die dem Fachmann hinlänglich bekannt sind, gelangt man zu Organismen, welche in der Lage sind, daß betrachtete Enzym in für einen technischen Prozeß ausreichender Menge zur Verfügung zu stellen. Die Herstellung der erfindungsgemäßen rec-Enzyme erfolgt nach dem Fachmann bekannten gentechnologischen Verfahren (Sambrook J, Fritsch EF, Maniatis T (1989). Molecular Cloning. Cold Spring Harbour Laboratory Press; Vectors: A Survey of Molecular Cloning Vectors and Their Uses. R.L. Rodriguez & D.T. Denhardt, Eds: 205-225). Bezüglich der allgemeinen Vorgehensweise (PCR und Fusions-PCR, inverse PCR, Klonierung, Expression etc.) sei auf folgende Literatur und das dort zitierte verwiesen: Riley J, Butler R, Finniear R, Jenner D, Powell S, Anand R, Smith JC, Markham AF (1990). A novel, rapid method for the isolation of terminal sequences from yeast artificial chromosome (YAC) clones. Nucl Acids Res. 18, 8186; Triglia T, Peterson MG, Kemp DJ (1988). A procedure for in vitro amplification of DNA segments that lie outside the boundaries of known sequences. Nucleic Acids Res. 16, 8186; Sambrook J, Fritsch EF, Maniatis T (1989). Molecular Cloning. Cold Spring Harbour Laboratory Press; Vectors: A Survey of Molecular Cloning Vectors and Their Uses. R.L. Rodriguez & D.T. Denhardt, II).

Wie schon angedeutet können die erfindungsgemäßen Nukleinsäuren auch zur Erzeugung neuer Mutanten der vorliegenden Hydantoinase benutzt werden. Solche Mutanten können durch bekannte Mutationsarten aus der erfindungsgemäßen DNA gewonnen werden. Bevorzugt anzuwendende Mutationsarten sind in folgenden Literaturstellen erwähnt: (Eigen M. and Gardinger W. (1984) Evolutionary molecular engineering based on RNA replication. Pure & Appl. Chem. 56(8), 967-978; Chen & Arnold (1991) Enzyme engineering for nonaqueous solvents: random mutagenesis to enhance activity of subtilisin E in polar organic media. Bio/Technology 9, 1073-1077; Horwitz, M. And L. Loeb (1986) "Promoters Selected From Random DNA-Sequences" Proceedings Of The National Academy Of Sciences Of The United States Of America 83(19): 7405-7409; Dube, D. And L. Loeb (1989) "Mutants Generated By The Insertion Of Random Oligonucleotides Into The Active-Site Of The Beta-Lactamase Gene" Biochemistry 28(14): 5703-5707; Stemmer PC (1994). Rapid evolution of a protein *in vitro* by DNA shuffling. Nature. 370; 389-391 und Stemmer PC (1994) DNA shuffling by random fragmentation and reassembly: *In vitro* recombination for molecular evolution. Proc Natl Acad Sci USA. 91; 10747-10751).

Mit 43 % Identität der Aminosäuren weist das Gen für die erfindungsgemäße Hydantoinase die höchste Homologie zu einem hypothetischen Protein aus Streptomyces coelicolor auf (T28685), dem bisher aber noch keine Funktion zugeordnet werden konnte. Zu den Dihydropyrimidinasen aus Bacillus stearothermophilus (JC2310: Mukohara et al., 1994), Agrobacterium radiobacter NRRLB11291 (Q44184: Grifantini et al., 1996) und Pseudomonas (Stover et al. 2000, La Pointe et al. 1998) liegen Identitäten von 40 % , 42 % und 39 % vor.

Neben den Homologien zu eukaryontischen Dihydropyrimidinasen (aus Mus musculus, Homo sapiens und Rattus norvegicus) und dem Collapsin response mediator Protein 3 (CRMP-3), liegen auch Homologien zu verschiedenen Allantoinasen und Dihydroorotasen vor.

Gegenüber den L-Hydantoinasen aus *Arthrobacter aurescens* DSM 3745 (May et al., Biol. Chem. 1998, 379, 743-747) und DSM 3747 (Wiese, Dissertation Universität Stuttgart, 2000) liegt eine 29 %-ige Identität vor.

Unter optisch angereicherten (enantiomerenangereicherten, enantiomer angereicherten) Verbindungen wird im Rahmen der Erfindung das Vorliegen einer optischen Antipode im Gemisch mit der anderen in >50 mol-% verstanden.

Unter Hydantoinen sind die sich aus 2,4-Dioxoimidazolidinen ableitenden Verbindungen gemeint, welche in 5-Stellung durch einen Rest substituiert sind, der sich von dem α-Rest einer Aminosäuren ableiten läßt.

Unter α-Rest einer Aminosäure wird der am α-C-Atom einer α-Aminosäure befindliche Rest verstanden. Dieser kann sich von einer natürlichen Aminosäure, wie in Beyer-Walter, Lehrbuch der organischen Chemie, S. Hirzel Verlag Stuttgart, 22. Auflage, 1991, S.822f. dargestellt, ableiten. Darüberhinaus sind jedoch auch entsprechende α-Reste unnatürlicher α-Aminosäuren gemeint wie z.B. in DE19903268.8 aufgeführt.

Der Organismus Arthrobacter crystallopoietes DSM 20117 ist bei der Deutschen Sammlung für Mirkroorganismen und Zellkulturen unter der entsprechenden Nummer hinterlegt und öffentlich zugänglich.

Der Begriff "aktiviert" oder "Aktivierung" soll anspruchsgemäß so verstanden werden, daß das erfindungsgemäße rec-Enzym gegenüber dem nicht aktivierten rec-Enzym bei gleicher OD₆₀₀ eine um den Faktor von mindestens 1,5, vorzugsweise 2, besonders bevorzugt 5 erhöhte Aktivität (Meßbedingungen gemäß Beispiel VI) im Zellextrakt (Überstand nach 15000 p.s.i., 60 sec., Zentrifugation bei 10000 r.p.m. für 10 min bei 4°C) besitzt.

Unter dem Begriff Nukleinsäuren werden alle Arten von einzelsträngiger oder doppelsträngiger DNA als auch RNA oder Gemische derselben subsumiert.

Unter verbesserten rec-Hydantoinasen werden anspruchsgemäß insbesondere solche verstanden, die ein modifiziertes Substratspektrum aufweisen, aktiver und/oder selektiver oder unter den verwendeten Reaktionsbedingungen stabiler sind.

Von den beanspruchten Proteinsequenzen und den Nukleinsäuresequenzen werden erfindungsgemäß auch solche Sequenzen umfaßt, die eine Homologie (exclusive der natürlichen Degeneration) größer als 80 %, bevorzugt größer als 90 %, 91 %, 92 %, 93 % oder 94 %, mehr bevorzugt größer als 95 % oder 96 % und besonders bevorzugt größer als 97 %, 98 % oder 99 % zu einer dieser Sequenzen aufweisen, sofern die Wirkungsweise bzw. Zweck einer solchen Sequenz erhalten bleibt. Der Ausdruck "Homologie" (oder Identität) wie hierin verwendet, kann durch die Gleichung H (%) = [1 - V/X] x 100 definiert werden, worin H Homologie bedeutet, X die Gesamtzahl an Nukleobasen/Aminosäuren der Vergleichssequenz ist und V die Anzahl an unterschiedlichen Nukleobasen/Aminosäuren der zu betrachtenden Sequenz bezogen auf die Vergleichssequenz ist.

### Beispiele:

### I. Biomassegewinnung von Arthrobacter crystallopoietes DSM 20117

Als Ausgangsmaterial für Ganzzellaktivitätstests, für die Isolierung chromosomaler DNA und zur Enzymisolierung der D-Hydantoinase sollte zunächst eine physiologisch einheitliche Zellmasse von Arthrobacter crystallopoietes DSM 20117 in ausreichender Menge bereitgestellt werden. Nach den Arbeiten von Brans (Doktorarbeit, TU Braunschweig, 1991) wurde dafür ein halbsynthetisches Medium mit D,L-Lactat als Kohlenstoffquelle, Hefeextrakt als weiterem Bestandteil und Hydantoin als Induktor für die Kultivierung im 50 Liter-Bioreaktor verwendet:

**Tabelle 1:**

| **Die Daten beziehen sich auf 1 l Nährlösung** | | |
|---|---|---|
| Natriumlactat- Medium pH 7,2 (V = 1 Liter) | Zitronensäure | 0,75 g |
| (Brans, 1991) | Hefeextrakt | 1,0 g |
| | FeSO₄ * 7 H₂O | 0,01 g |
| | MgSO₄ * 7 H₂O | 0,5 g |
| | CaSO₄ * 2 H₂O | 0,22 g |
| | MnSO₄ * H₂O | 0,055 g |
| | ZnSO₄ * 7 H₂O | 0,005 g |
| | (NH₄)₂SO₄ | 6,0 g |
| | D,L-Methionin | 0,05 g |
| | Hydantoin | 1,0 g |
| | 50 %-iges D,L-Lactat | 40 ml |
| | 1 M KH₂PO4 | 23 ml |

Eine erste Vorkultur (V = 20 ml) wurde über Nacht bei 30 °C und 110 rpm inkubiert. Anschließend wurde die gesamte Vorkultur zum Animpfen der zweiten Vorkultur (V = 2 l) verwendet. Nach zwei Tagen Inkubation wurden 1,5 l der zweiten Vorkultur als Inokulum für die Fermentation (V = 20 l) genutzt. Da der Induktor Hydantoin während des Wachstums verbraucht wird, wurde dieses mit einer Förderpumpe kontinuierlich zudosiert, so das die Hydantoinkonzentration im Medium konstant 0,2 g/l betrug. Nach der Zellernte wurden 205 g BFM aliquotiert und bei - 20 °C gelagert.

### II. Aufreinigung der D-Hydantoinase aus Arthrobacter crystallopoietes DSM 20117

Das Protokoll zur Aufreinigung der D-Hydantoinase aus Arthrobacter crystallopoietes DSM 20117 orientiert sich mit einigen Modifikationen an der von Marin (Doktorarbeit, Uni Stuttgart, 1997) beschriebenen Proteinaufreinigung der D-Hydantoinase. Die Aufreinigungschritte wurden, wenn möglich, bei 4 °C durchgeführt und die Bestimmung der Hydantoinaseaktivität der Fraktionen erfolgte zunächst im Schnelltest mit dem photometrischen Nachweis nach Ehrlich. Aliquots der positiven Proben wurden anschließend mit dem Standardsubstrat D,L-Benzylhydantoin inkubiert und die exakte Aktivität mittels HPLC bestimmt.

Die aus der Kultivierung erhaltene Biomasse (siehe I) von Arthrobacter crystallopoietes DSM 20117 wurde zunächst als 30 %-ige Zellsuspension einem Glasperlenaufschluß in der Rührwerkskugelmühle unterzogen. Nach der Aufnahme einer Aufschlußkinetik, konnten nach 20 minütiger Aufschlußzeit Proteinkonzentrationen von bis zu 16,5 g/l erreicht werden. Danach wurden die Zelltrümmer sowie unlösliche Bestandteile durch Zentrifugation abgetrennt und der geklärte Überstand für die folgende Protaminsulfatfällung eingesetzt. Mit dieser ließ sich vor Durchführung einer Streamline-DEAE-Säulenchromatographie die Viskosität der Lösung verringern.

Die auf der Säule gebundenen Proteine wurden mittels eines Kochsalzgradienten eluiert. Die aktiven, gepoolten Streamlinefraktionen wurden mit einem gleichen Volumen 2 M (NH₄)₂SO₄-Lösung versetzt, um sie anschließend mittels hydrophober Interaktionschromatographie (HIC) weiter aufzutrennen. Die Fraktionen mit der höchsten Hydantoinaseaktivität wurden anschließend vereinigt und über Anionenaustauschchromatographie an einer MonoQ-Säule von anderen Proteinen getrennt.

Die Daten zur Aufreinigung der Hydantoinase sind in Tabelle 2 zusammengefaßt, die SDS-PAGE der aufgereinigten D-Hydantoinase ergab ein Molekulargewicht von 50 +/- 5 kDa für dieses Enzym [10 %-ige SDS-PAGE der aufgereinigten D-Hydantoinase nach Konzentrierung der MonoQ-Fraktionen, Molekulargewichtsmarker ProSieve und L-Hydantoinase aus A. aurescens DSM 3745 als interner Standard von 49,7 kDa (May, Dissertation Uni Stuttgart, 1998)].

**Tabelle 2:**

| **Aufreinigungsdaten für die D-Hydantoinase** | | | | | |
|---|---|---|---|---|---|
| Reinigung | Vol. [ml] | Prot. [g/l] | spez. Akt. [U/mg] | Reinigungsfaktor | Ausbeute [%] |
| Zellaufschluß | 32 | 16 | 1,5 | - | 100 |
| Protaminsulfatfällung | 29 | 17 | 1,4 | 0,9 | 89 |
| vereinigte Streamlinefrakt. | 61 | 3,8 | 1,9 | 1,3 | 57 |
| Überstand Ammoniumsulfatfällg. | 120 | 1,5 | 3,7 | 2,4 | 85 |
| vereinigte HIC-Fraktionen | 30 | 0,8 | 13,3 | 8,8 | 41 |
| vereinigte MonoQ-Fraktionen | 19 | 0,4 | 30,1 | 19,8 | 29 |

### III. Tryptischer Verdau der D-Hydantoinase

N-terminale Sequenzierungen liefern sichere Sequenzergebnisse nur für die ersten 30 Aminosäuren. Die in der Arbeit von Marin angegebene Sequenz ließ eine Ableitung von Primern allerdings nicht zu. Daher mußte das Protein zur weiteren Sequenzinformation durch einen Proteaseverdau in mehrere Peptide zerteilt werden. Zur enzymatischen Fragmentierung wurde mit Trypsin eine Endopeptidase verwendet, die spezifisch nach den Aminosäuren Lysin und Arginin schneidet. Allerdings ist mit einer verminderten Aktivität zu rechnen, wenn eine saure Aminosäure folgt, und sogar mit einem Ausbleiben der Hydrolyse, wenn ein Prolinrest folgt. Bei einem durchschnittlichen Vorkommen von Lysin und Arginin von 5,7 % bzw. 5,4 % in Proteinen, ist bei vollständigem Verdau mit einer durchschnittlichen Peptidlänge von etwa 9 Aminosäuren zu rechnen. Die Auftrennung des Peptidgemisches erfolgte anschließend durch quantitative HPLC.

Um die Hydantoinase aus Arthrobacter crystallopoietes DSM 20117 mit Trypsin zu verdauen, wurde diese wie beschrieben bis zu den MonoQ-Fraktionen aufgereinigt, anschließend mit einem Amicon-Filter (cut-off 30 kDa) aufkonzentriert und mittels SDS-PAGE aufgetrennt. Um sicher zu gehen, dass es sich bei dem Protein auch um die D-Hydantoinase handelte, wurde ein Teil des Geles über einen Western-Blot auf eine Membran transferiert, ausgeschnitten und N-terminal die ersten acht Aminosäuren bestimmt. Mit Ausnahme von Position 2 stimmten alle ermittelten Aminosäuren mit dem von Marin (Dissertation, Universität Stuttgart, 1997) bestimmten N-Terminus überein, sodass davon ausgegangen werden konnte, dass es sich bei dem hier isolierten Protein um dasselbe Enzym handelte, das von Marin bereits beschrieben und charakterisiert wurde.

Daraufhin wurde die Hydantoinase-Bande direkt aus dem Polyacrylamidgel der aufgetrennten MonoQ-Fraktionen ausgeschnitten und in situ nach Angaben des Herstellers (Sigma, Steinheim) tryptisch verdaut. Die Peptide wurden mit Acetonitril aus dem Gel extrahiert und mittels präperativer HPLC voneinander getrennt. Die Fraktionen wurden im Speed-vac eingetrocknet und anschließend N-terminal über Edman-Abbau sequenziert.

Insgesamt konnten zusätzlich zum N-Terminus neun Peptide eindeutig sequenziert werden. Eines der Peptidfragmente wies das Konsensusmotiv GXXDXHXH der cyclischen Amidasen auf, das an der Bindung eines Zinkatoms im aktiven Zentrum beteiligt ist (Abendroth et al., Acta Cryst. 2000, D56, 1166-1169). Bei den Peptidsequenzen, die nicht mit einem Lysin (K) oder Arginin (R) enden, brach die Sequenzierung aufgrund technischer Probleme oder mangelnder Qualität bzw. Quantität der Proben frühzeitig ab.

### IV. Klonierung des hyu-Genclusters

### 1. Isolierung chromosomaler DNA aus Arthrobacter crystallopoietes DSM 20117

Die durch Kultivierung von Arthrobacter crystallopoietes DSM 20117 auf Lactatmedium gewonnene Biofeuchtmasse (siehe I) diente auch zur Isolierung chromosomaler DNA. Nach Zelllysis und Aufreinigung mittels Cäsiumchlorid-Dichtegradientenzentrifugation konnte hochreine, genomische DNA isoliert werden. Die Qualität wurde durch Aufnahme eines Absorptionsspektrum geprüft, um auf diese Weise Kontaminationen mit Phenol ausschliessen zu können. Die photometrisch bestimmte DNA-Konzentration betrug 60 µg DNA/ml.

Die cDNA wurde für einen Restriktionsverdau eingesetzt und als Matrize für PCRs verwendet.

### 2. PCR mit degenerierten Primern

Durch die Sequenzierung der aus dem tryptischen Verdau hervorgegangenen Peptide (siehe III) konnten zusätzlich zum N-Terminus der D-Hydantoinase weitere Sequenzinformationen gewonnen werden. Die Peptide wurden mit dem Programm ClustalX an die bekannte Proteinsequenz von Agrobacterium sp. IP I-671 angepaßt (Thompson et al. 1997, The ClustalX windows interface: flexible strategies for multiple sequence alignment aided by quality analysis tools. Nucleic Acids Research. 24, 4876-4882).

Um von den bekannten Peptidsequenzen degenerierte Primer abzuleiten, sollten Sequenzabschnitte von zwei Peptiden ausgewählt werden, die einen niedrigen Degenerierungsgrad in der Aminosäurezusammensetzung besitzen. Hierfür wurden die Peptide 61.61 und 73.31 ausgwählt. Der Primer 61.61a paart an den Plusstrang und der Primer 73.31b an den Minusstrang der DNA.

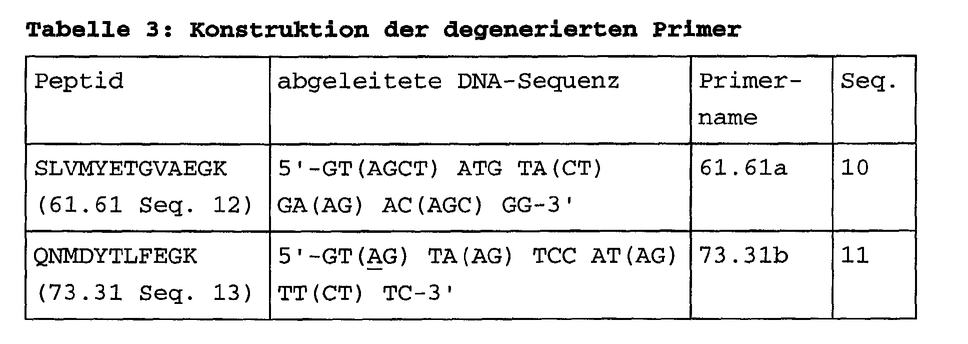

Um den Degenerierungsgrad des Primers 61.61a weiter zu reduzieren, wurde auf Basis der Datenbank CUTG die Häufigkeitsverteilung der Codons aus Arthrobacter sp. berücksichtigt (Nakamura et al., Nucl. Acids Res. 1999, 27, 292). Dadurch konnte an der Position 3 dieses Oligonucleotides das Basentriplett "GTA", aufgrund der niedrigen Wahrscheinlichkeit dieses Codons von 10,4 % für die Aminosäure Valin, bei der Primerkonstruktion vernachlässigt werden.

Um die Länge des PCR-Amplikons abzuschätzen, wurde ein Alignment der beiden Primer an die D-Hydantoinase aus Agrobacterium sp. IP I-671 durchgeführt. Im Alignment beträgt der Abstand zwischen den beiden Oligos 69 Aminosäuren, sodass eine PCR mit den degenerierten Primern 61.61a und 73.31b zu einem PCR-Produkt von ca. 207 bp Länge führen sollte.

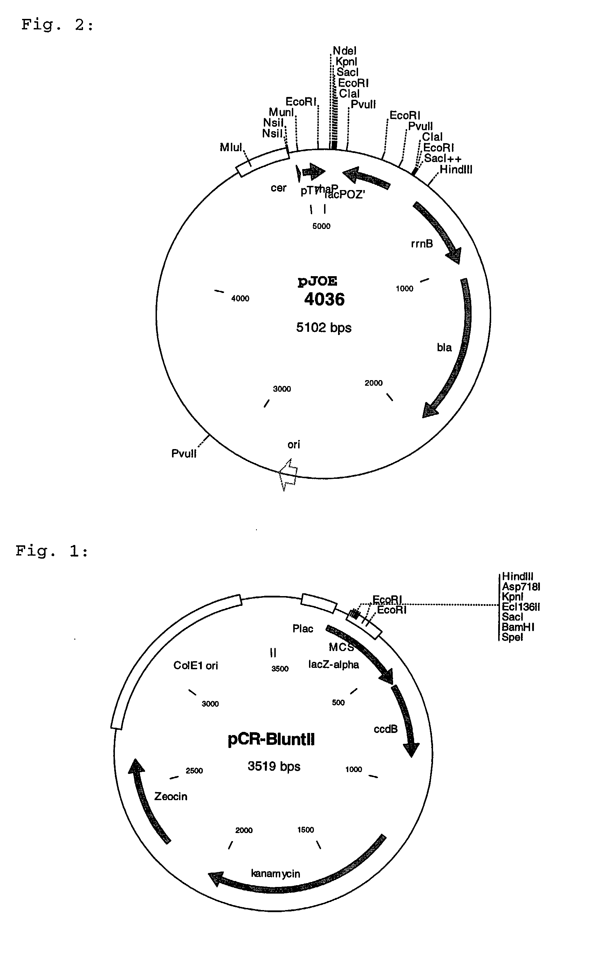

Die PCR wurde im Temperaturprofil nach dem Standardardansatz bei einer Annealingtemperatur von 42 °C angesetzt und bezüglich des Magnesiumgehaltes auf eine Konzentration von 2 mM optimiert. Der PCR-Ansatz wurde anschließend in einem 3 %-igen Agarosegel aufgetrennt und die Größe der Banden mit der Bildanalysesoftware Imagemaster bestimmt (Molekulargewichtsmarker D-15 Firma Novex). Die Bande, die eine berechnete Größe von 218 bp besaß, wurde aus dem Gel eluiert und in den pCR TOPO BluntII-Vektor (Fig. 1) ligiert. Das erhaltene Plasmid wurde als pJW1 bezeichnet. Eine anschließende Sequenzierung des Vektors ergab Homologien zu bereits bekannten Dihydropyrimidinasen, sodass damit der erste DNA-Abschnitt auf dem Strukturgen der D-Hydantoinase kloniert vorlag.

### 3. Sequenzierung des hyu-Genclusters über Inverse PCR

Um weitere Sequenzinformationen von den flankierenden DNA-Bereichen zu erhalten, kam die Technik der inversen PCR (IPCR) zum Einsatz.

Zum Verdau genomischer DNA aus Arthrobacter crystallopoietes DSM 20117 fanden die Restriktionsenzyme *Bam*HI, *Eco*RI, *Sac*I, *Pst*I, *Bgl*II, *Hind*III, *Sal*I, *Mun*I, und *Mlu*I Anwendung. Die Verdaue wurden über ein 1 %-iges Agarosegel aufgetrennt und mittels Southern-Blot auf eine Nylonmembran fixiert.

Zur Herstellung einer geeigneten Sonde wurde das *Mun*I linearisierte Plasmid pJW1 über Nick-Translation (Nick-Translation Kit der Firma Roche Diagnostics) mit ³²P-α-ATP radioaktiv markiert und zur Hybridisierung mit dem Blot eingesetzt (Molekulargewichtsmarker MWM VII).

Aufgrund der aus dem Southern-Blot erhaltenen Größe der Hybridisierungssignale wurde bei der folgenden IPCR der genomische *Pst*I-Verdau (ca. 2000 bp) als Matrize eingesetzt. Dazu wurde der Verdau auf einem Agarosegel aufgetrennt, im Bereich zwischen 1500 und 2800 bp aus dem Gel eluiert (Molekulargewichtsmarker MWM VII), anschließend religiert und mit *Mun*I linearisiert. Aus der bekannten Sequenz des Hydantoinasegens konnten die Primer IPCR1+ (Seq. 3) und IPCR1- (Seq. 4) für die IPCR abgeleitet werden. Aus den Schmelztemperaturen der Oligos leitete sich die Annealingtemperatur von 60 °C ab.

Es konnte eine einzige Bande als Amplikon generiert werden, die anschließend eluiert und in das TOPO-System (Fig. 1) kloniert wurde. Das entstandene Plasmid erhielt die Bezeichnung pJW2. Das nach der Sequenzierungen von pJW2 rekonstruierte *hyu*-Gencluster enthält den offenen Leserahmen der D-Hydantoinase *hyuH* und einen Teil des offenen Leserahmens der D-Carbamoylase *hyuC*_{*D*}.

### V. Expression der D-Hydantoinase

Die Klonierung der Strukturgene der D-Hydantoinase erfolgte in Plasmidderivaten des Rhamnoseexpressionsvektors pJOE4036 (Fig. 2). Die beiden Carbamoylasen wurden durch entsprechende Primer aus der genomischen DNA von *Arthrobacter crystallopoietes* amplifiziert. Dabei waren die Primer am N-Terminus mit einer zusätzlichen Sequenz für eine *Nde*I-Schnittstelle bzw. eine *Bam*HI-Schnittstelle am C-Terminus ausgestattet. Bei den Enzymen mit His-Tag wurde am C-terminalen Primer das Stopcodon weggelassen.

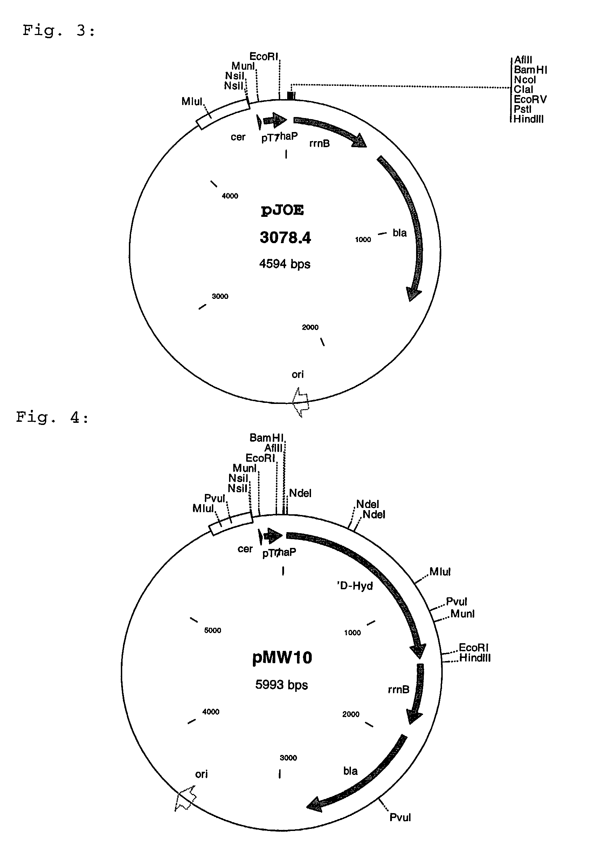

Weil das Hydantoinasegen zwei interne *Nde*I-Schnittstellen aufwies, konnte die bei den Carbamoylasen angewandte Strategie der Klonierung in pJOE4036 nicht angewandt werden. Statt dessen verwendete man einen Primer, der zusätzlich zur N-terminalen DNA-Sequenz am 5'-Ende für eine Shine-Dalgarno-Sequenz und eine *Bam*HI-Schnittstelle codierte. Der C-terminale Primer beinhaltete eine *Hind*III-Schnittstelle mit Stopcodon bzw. mit einer Sequenz für einen Strep-tag. Die so aus der genomischer DNA amplifizierte DNA wurde anschließend in pJOE3078 kloniert. Das entstehende Konstrukt wurde pMW10 (Fig. 4) genannt.

Ein E. coli enthaltend das Plasmid pMW10 (Fig. 4) wurde in LB-Medium, welches 100 µg/ml Ampicillin enthielt, wie folgt angezogen:
- eine einzelne Kolonnie wurde in 10 ml LB-Medium in einen 100 ml Schüttelkolben transferiert und bei 37°C über Nacht inkubiert
- 100 ml des LB-Mediums mit der angegebenen Menge Ampicillin wurde in einem 500 ml Schüttelkolben mit 2 ml der über Nacht inkubierten Kultur versetzt (Ansatz 1).
- In einem zweiten Schüttelkolben (500 ml) wurde eine gleiche Menge an Übernachtkultur, LB-Medium und Ampicillin angerichtet und zusätzlich mit 1 ml einer 100 mM ZnSO₄*7H₂O-Lösung versetzt (Zn²⁺-Konz. in der Kultur ImM) (Ansatz 2).
- Ansatz 1 wurde 2 h bei 37°C inkubiert bis die OD₆₀₀ ∼0.4 betrug. Ansatz 2 brauchte für den selben OD-Wert 3 h.
- Zur Induktion der Expression wurden beide Ansätze mit L-Rhamnose versetzt, bis eine Konzentration von 0.1 g/L erreicht war. Anschließend kultivierte man die Ansätze weitere 6 h bei 30°C.
- Die Zellen wurde anschließend für 10 min bei 4°C und 7000 r.p.m zentrifugiert. Die erhaltenen Pellets wurden bei -10°C aufbewahrt.

### VI. Messung der Aktivität

1 g der aufbewahrten Zellen aus V. wurde jeweils in 10 mL 0.1 M Phosphat-Puffer (pH 7.5) resuspendiert. Die Zellen wurden anschließend bei 15000 psi innerhalb von 60 sec aufgeschlossen und die erhaltenen Suspensionen bei 10000 r.p.m. für 10 min bei 4°C zentrifugiert. 25 µl des Überstandes wurden jeweils in eine vorgewärmte Probe von 100 µL 0.1 M Phosphat-Puffer (pH 7.5) enthaltend 8 mM D-Benzylhydantoin gegeben. Die Reaktion wurde nach Inkubation der Proben für 5 min bei 50°C durch Zugabe von 100 µl 10% H₃PO₄ gestoppt. Nach erneuter Zentrifugation bei 10000 r.p.m. für 10 min wurden jeweils 100 µl des Überstandes 10-fach mit der mobilen Phase des HPLC-Laufmittels verdünnt (0.3%(v/v) Phosphorsäure (80%), Methanol (20%v/v)). Mittels HPLC wurden dann die Konzentrationen der entstandenen N-Carbamoylaminosäuren bestimmt.
- HPLC-Methode:: Thermoseparation products, Darmstadt, Germany Gromsil ODS 1 PE-Säule (5 µm, 250x4.6 mm, Grom, Herremberg, Germany) UV-Adsorption bei 210 nm 1.0 mL/min Flußrate

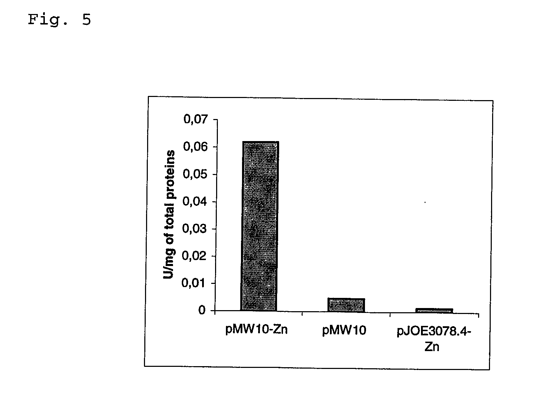

Die spezifische Aktivität ist definiert in Einheiten pro mg des totalen Proteins bestimmt nach Bradford. Eine Einheit der D-Hydantoinase katalysiert die Bildung von 1 µmol Carbamoylaminosäure ausgehend von D-Benzylhydantoin pro min bei pH 7.5 und 50°C. Fig. 5 zeigt das Ergebnis der spezifischen Aktivität der D-Hyd im Zellextrakt für beide Ansätze verglichen mit dem das hyd-Gen nicht enthaltenden Mikroorganismus.

Es ist zu sehen, daß die in Gegenwart von erhöhten Zink-Konzentrationen fermentierte Probe eine um den Faktor >12 aktivere Hydantoinase enthält.

## Patentansprüche

1. Verfahren zur Herstellung aktivierter rec-Hydantoinasen durch Fermentation der die rec-Hydantoinasen bildenden Mikroorganismen in Gegenwart zweiwertiger Metallionen,
**dadurch gekennzeichnet, daß**
die Fermentationsbrühe eine die Aktivierung bedingende Konzentration an diesen Metallionen aufweist.

2. Verfahren nach Anspruch 1,
**dadurch gekennzeichnet, daß**
die Konzentration an Metallionen, insbesondere Zinkionen bei ≥30 µmol/l liegt.

3. Verfahren nach Anspruch 1,
**dadurch gekennzeichnet, daß**
es sich bei der rec-Hydantoinase um die Hydantoinase aus Arthrobacter crystallopoietes DSM20117 handelt.

4. rec-Hydantoinase erhältlich nach einem Verfahren gemäß Anspruch 1.

5. Nukleinsäuren codierend für eine D-Hydantoinase aus Arthrobacter crystallopoietes DSM 20117.

6. Plasmide, Vektoren, Mikroorganismen aufweisend eine oder mehrere Nukleinsäuren nach Anspruch 5.

7. Nukleinsäuren hybridisierend mit den einzelsträngigen Nukleinsäuren oder komplementären einzelsträngigen Nukleinsäuren gemäß Anspruch 5 unter stringenten Bedingungen.

8. Primer zur Herstellung der Nukleinsäuren nach Anspruch 5 mittels PCR.

9. Verfahren zur Herstellung von verbesserten rec-Hydantoinasen ausgehend von Nukleinsäuren codierend für eine rec-Hydantoinase nach Anspruch 4,
**dadurch gekennzeichnet, daß** man
a) die Nukleinsäuren einer Mutagenese unterwirft,
b) die aus a) erhältlichen Nukleinsäuren in einen geeigneten Vektor kloniert und diesen in ein geeignetes Expressionsystem transferiert und
c) die gebildeten Proteine mit verbesserter Aktivität und/oder Selektivität detektiert und isoliert.

10. rec-Hydantoinasen oder diese codierende Nukleinsäuren erhältlich nach Anspruch 9.

11. Verwendung der rec-Hydantoinasen gemäß Anspruch 4 oder 10 zur Herstellung von N-Carbamoylaminosäuren bzw. Aminosäuren.

12. Verwendung der Nukleinsäuren gemäß Anspruch 5 oder 10 zur Herstellung von Ganzzellkatalysatoren.
